# EUROPEAN PATENT SPECIFICATION

(11) **EP 0 883 614 B1**
(45) Date of publication and mention of the grant of the patent: **24.10.2001**
(21) Application number: 96940675.0
(22) Date of filing: 12.12.1996
(51) Int. Cl.: C07D 307/60

(54) **ADDITIVES USABLE IN PREPARATION OF ALKENYL SUCCINIC ANHYDRIDE**
NÜTZLICHE ADDITIVE IN DER HERSTELLUNG VON ALKENYLBERNSTEINSÄUREANHYDRID
ADDITIF POUR LA PREPARATION D'ANHYDRIDE D'ALCENYLE SUCCINIQUE

(30) Priority: 13.02.1996 FI 960639
(43) Date of publication of application: 16.12.1998
(73) Proprietor: KEMIRA CHEMICALS OY, 00100 Helsinki (FI)
(72) Inventor: MÄKIPEURA, Petri, FIN-06850 Kulloo (FI); KAPANEN, Mika, FIN-06100 Porvoo (FI); TULISALO, Jukka, FIN-04230 Kerava (FI); KOSKIMIES, Salme, FIN-00850 Helsinki (FI)
(74) Representative: Hansen, Bernd, Dr. Dipl.-Chem.
(86) International application number: FI9600656
(87) International publication number: WO9730039

(56) References cited:
- US-A- 3 412 111
- US-A- 4 956 478
- CHEMICAL ABSTRACTS, Volume 97, No. 2, 12 July 1982, (Columbus, Ohio, USA), page 34, Abstract No. 7229t; & JP,A,57 032 276 (DAINIPPON), 20 February 1982.
- CHEMICAL ABSTRACTS, Volume 107, No. 26, 28 December 1987, (Columbus, Ohio, USA), page 119, Abstract No. 238905b; & JP,A,62 106 091 (KOKAI TOKKYO KOHO), 16 May 1987.

## Description

The present invention relates to a process for the preparation of an alkenyl succinic anhydride from an olefinically unsaturated hydrocarbon and a maleic anhydride by using additives which improve the yield and reduce side reactions.

Alkenyl succinic anhydrides, or ASAs, are reaction products of olefins and maleic anhydride (MHA). They are produced by the ene reaction, which is an indirect substituting addition from a compound with an electron-poor double bond (enophil) to a compound which contains an allylic hydrogen (ene). In the preparation of ASA, the enophil component is MHA and the ene component is olefin. In the reaction, the double bond of the olefin shifts to the subsequent carbon, a new bond is formed between the olefin and MHA, and the allylic hydrogen present in the olefin shifts to MHA.

The ene reaction requires a relatively high temperature, since its activation energy is high, i.e. approx. 20 kcal/mol. The reaction speed of the reaction also increases strongly as a function of the temperature, and thus it is preferable to use a relatively high reaction temperature in order for the product to form at a reaction speed which is at least satisfactory. For the above reasons, ASA is produced at relatively high temperatures, and thereby also the probability of the occurrence of various side reactions increases. Side reactions observed in the preparation of ASA include the polymerization of MHA, the copolymerization of olefin and MHA, and the oligomerization of olefin. It has also been observed in the preparation of ASA that further reactions of ASA occur, which include further reaction of ASA with MHA and the retroene reaction, in which ASA breaks down to its initial substances. Thermal decomposition of MHA and ASA can also be observed in the preparation of ASA. The side and decomposition reactions lead to a poor yield and a poor quality of the actual product, i.e. the forming product often has a strong color and contains large amounts of various polymeric and tarry compounds.

Attempts are made, by using various additives, to reduce the side reactions occurring in the preparation. The purpose of the additives is to lead to a purer and more monomeric product having less color. This is done for the reason that ASAs often have a high molecular weight, and in practice they are impossible to purify, for example, by distillation. The additives may work through various mechanisms, for example through the catalysis of the principal reaction or the inhibition of the side reactions but, with respect to many of the additives used in the preparation of ASA, the way in which the additive works is not known with full certainty.

Alkenyl succinic anhydrides may be prepared from olefins of highly different lengths, ranging from propylene to polymers having hundreds of carbon atoms. ASAs prepared from polymers, mainly polyisobutene, are used as dispersants and corrosion inhibitors in additives of diesel and gasoline motors. The molecular weight of the polymer used usually ranges from 900 to 1500 g/mol, and the polymer is prepared from isobutene.

ASA may also be prepared from shorter-chain olefins, as pointed out above. Of shorter-chain olefins, mainly 16-20 carbon atom α-olefins are used, which may have been isomerized before the preparation of ASA. In addition, C₆-C₁₄ α-olefins and C₁₅-C₁₈ internal olefins are used on a larger scale. ASAs prepared from these olefins are used mostly as reactive paper-sizing chemicals. The ASAs used for paper sizing are prepared from C₁₆-C₂₀ α-olefins, which are isomerized, and thus an ASA which is liquid at room temperature is obtained. In paper making, the anhydride group present in ASA reacts with the hydroxyl group of cellulose, and the alkenyl chain forms on the surface of paper or cardboard a hydrophobic surface which prevents water and ink from penetrating into the paper. The amount of ASA used in paper sizing will increase, since in paper making a shift is being made to paper making in alkaline conditions, in which conventional hydrophobic sizes cannot be used.

Starch derivatives of ASA, prepared from even shorter-chain olefins, such as octenes, are used as coagulants in foods, juices and puddings. Sodium and potassium salts of octenyl succinic anhydride for their part are used as surfactants in industrial metal-cleaning and floor-cleaning agents. ASAs made from α-C₁₂-C₁₈ are used as leather-finishing substances to soften leather and to protect it from water. ASAs made from these olefins may also be used as auxiliaries in epoxy resin setting agents, whereby better electrical and flexibility properties are obtained for the resin, and as additives of oil and as corrosion inhibitors. ASA can also be used for preparing various ester, amide, imide, and other derivatives, which are used partly in applications similar to those in which actual ASA is used, for example as additives in oil and as corrosion inhibitors.

ASAs are prepared by the ene reaction from olefins and maleic anhydride. The reaction requires a high temperature, 150-300 °C. Side reactions occurring at this temperature include polymerization of MHA, thermal decomposition of MHA, copolymerization of MHA and olefin, and oligomerization of olefin. The side reactions cause the efficiency of the reaction to deteriorate, which in turn leads to a poor yield of ASA and an impure product. Below, there are presented various additives used in the preparation of ASA in order to inhibit side reactions.

According to patent publications US 3 412 111 and EP 359 316, attempts have been made to reduce polymerization in the preparation reactions by using hydroxy or aminoaromatic compounds as additives in the preparation of ASA. The initial olefin used in the examples of the said patent publications is dodecene or a mixture of dodecene and tetradecene. In patent publication US 3 476 774, sterically hindered phenols, such as 2,6-di-t-butyl-4-methylphenol (BHT), are used for reducing the breaking down of MHA. In the examples of the patent publication, ASAs were prepared from polyisobutene, and in some of the experiments an organic solvent, such as xylene, was also used. However, a good ASA yield is not obtained by using the additives used in these patent publications, and the product obtained contains varying amounts of polymeric compounds. Furthermore, a product of unsuitable color is obtained with some of the additives mentioned in the patent publications.

In patent publication US 4 599 433, the additives used in the preparation of ASA are alkoxides of Ti, Zr, V or Al, of which C₂-C₄ alkoxides would be recommendable. It has been observed that the additives mentioned in the patent publication increase the yield of ASA and reduce the polymerization of MHA. In the examples of the patent publication, ASA is prepared by using polyisobutene and 2,4,4-trimethylpentene, and as the additive titanium butoxide. ASAs prepared in accordance with the patent publication can, according to the publication, be used as anticorrosive agents and as additives in oil.

In patent publication US 4 761 488, aluminum acetylacetonate is used as an additive in the preparation of ASA to reduce the formation of a black solid, and it has been found to improve considerably the color of the ASA formed. The olefin used in the examples of the patent publication is tetradecene or C₁₅-C₂₀ internal olefins. ASAs prepared in accordance with the patent publication are used, according to the publication, as anticorrosive agents.

However, as later examples show (Comparative Examples 2, 3, 15), a high yield of ASA and a polymer-free product have not been achieved by using the additives according to the said patent publications US 4 599 433 and US 4 761 488.

When ASA is prepared in accordance with patent publication US 4 431 825 from olefins (mainly polyisobutenes having Mₙ = 300-3000), the preparation reaction can be catalyzed with iron or tin chlorides, iron bromide or phosphoric acid. However, the catalyzing effect of iron chloride was not observed in Examples 4 and 15. It is disclosed in patent publication US 3 935 249 that by using as additives inorganic compounds, preferably compounds containing chlorine or bromine, it is possible to reduce the formation of tarry byproducts which are formed because of polymerization, and to reduce the thermal decomposition of MHA. The problem involved with the additives according to these patent publications consists of various halogenic compounds, which may be environmentally hazardous, and, for example, chlorine is a highly corrosive chemical.

According to patent publication EP 317 004, various alkyl zincs and zinc alkoxides can be used as additives in the preparation of ASA in order to reduce side reactions appearing in the preparation. In the experiments in the examples of the patent publication, these additives did not improve the yield of ASA as compared with ASA prepared without the additive, only the color of the product improved somewhat. The olefins used in the examples of the patent publication were C₁₄-C₂₀ olefins, which may be either α-olefins or internal linear or branched olefins. According to the publication, ASAs prepared in accordance with the patent publication are used as anticorrosive agents.

In patent publications US 4 958 034 and US 5 021 169, aryl fluorophosphates and tri-orthoalkylphenyl phosphates are used as additives. In these patent publications, attention has been paid to the color of the product formed and to tar formation, but not to the yield with which ASA can be prepared. In the examples of both patent publications, ASA is prepared from C₁₆-C₁₈ α-olefins which have been rendered internal by isomerization. However, an ASA prepared with the additives according to the patent publication has contained polymeric compounds almost in an amount of 10 %, depending on the additive used.

In patent publication US 4 581 464, a C₈-C₁₈ alkyl succinic anhydride is used as an additive in the preparation of ASA. By means of this additive, the initial substances used in the reaction, i.e. MHA and α-olefin, have together been rendered a homogenous mixture, which reduces the formation of undesirable side reactions. The reaction mixture must contain alkyl succinic anhydride in an amount of at least 20 %. The use of this additive is uneconomical, since it is impossible to separate from the end product.

In patent publication US 4 388 471, furan-type compounds are used as an additive in the preparation of ASA. These compounds are believed to react with MHA through a reversible cycloaddition, the addition product then breaking down under heat. Thus the amount of free MHA in the reaction mixture is as small as possible, and this reduces the polymerization of MHA. But the amount of the necessary furan-type compound in the reaction mixture must be large in order to lead to the desired end result. The compound used must be removed before the end use of ASA, and thus it is uneconomical to use such additives.

In patent publication GB 1 337 724, aliphatic or aromatic organic phosphorus-containing compounds or orthophosphoric acid is used as one additive and hydroxyaromatic antioxidants as a second additive. The use of the compounds in accordance with the patent publication gives the product a light color, but the product contains polymeric compounds. The ASAs prepared in accordance with the patent publication are preferably prepared from C₆-C₂₀ α-olefins and are used as additives of polymers.

Patent publication GB 1 396 097 is an improved version of patent publication GB 1 337 724 cited above. In this latter publication there is used, in addition to the above additives, transition metal ions as a third additive. It has been found that they further improve the color of the product and somewhat increase the amount of monomeric product, but polymeric compounds still appear.

In all of the above-mentioned patents and processes there are various problems in the preparation of ASA. They often lead to a poor yield of the product, and the end products still contain large amounts of polymers, or the processes are in other respects unsuitable and uneconomical. We have now observed in our experiments, surprisingly, that when there is used as one additive in the preparation of ASA an aromatic compound which contains sulfur and nitrogen and as a second additive a phenolic antioxidant or its derivative, the amount of polymers in the product can be minimized, the product obtained is light in color, and the product is obtained with a very good yield as compared with an antioxidant alone or with other prior known methods.

It is characteristic of an aromatic compound which contains sulfur and nitrogen that it contains, linked to adjacent carbon atoms in the aromatic ring, sulfur and nitrogen atoms which for their part are linked to the same carbon atom or adjacent carbon atoms in some hydrocarbon group, for example, the adjacent carbon atoms in the second benzene ring. Examples of such compounds are thioamines which contain one or two benzene rings, such as benzothiazole and phenothiazine and derivatives of these. Thiodiphenylamine, i.e. phenothiazine, has been found to be a preferable compound.

ASA is prepared at a temperature of 150-250 °C by allowing an olefin and MHA to react with each other after an aromatic compound containing nitrogen and sulfur, for example phenothiazine or a phenolic antioxidant, has been added to the mixture in a catalytic amount. It is preferable to use a reaction temperature of 180-230 °C in order to increase the efficiency of the reaction. The reaction period may range from 0.5 to 24 hours. The ratio of olefin to MHA may vary within 0.1-5.0/1 (mol/mol), depending on the reaction temperature, reaction period and additive used. The amount of the additive mixture in the reaction mixture is for its part also dependent on the reaction temperature, reaction period and additive used, in such a way that each additive is added to the reaction mixture in an amount of 0.01-5.0 wt.% of the amount of MHA used. The ratio of the aromatic compound containing nitrogen and sulfur to the phenolic antioxidant may vary widely, i.e. the amount of aromatic compound containing nitrogen and sulfur, for example phenothiazine, may be 0.1-10 times the amount of phenolic antioxidant. In our experiments, an amount of approx. 0.25-2 g of phenothiazine per 1 g of phenolic antioxidant has proven to be advantageous. Finally any unreacted initial materials are removed from the reaction mixture by vacuum distillation, and a final product (ASA) which is so pure that it need not be purified before use is obtained with a good yield.

The additive mixture used in the reaction mixture thus contains two components, both an aromatic compound containing sulfur and nitrogen, preferably phenothiazine, and a phenolic antioxidant or a derivative thereof. The phenolic antioxidant or its derivative may be, for example, a mono- or dihydroxyaromate or a derivative thereof, such as phenol, cresol, catechol, hydroquinone, tert-butylhydroquinonediacetate, monomethyl ether of hydroquinone (i.e. 4-methoxyphenol), 2,6-di-tert-butyl-4-methylphenol, or 4,4'-methylenebis-(2,6-di-tert-butylphenol). Other phenolic antioxidants suitable for use together with an aromatic compound which contains sulfur and nitrogen are listed and described in patent publications US 3 412 111 and US 3 476 774.

The molar mass of the olefin used for the preparation of ASA may vary within 36-500 g/mol, depending on the intended use of the ASA obtained as the end product. The structure of the olefin used in the preparation is also dependent on the intended end use.

The olefin used in the preparation may be by structure a straight-chain α-olefin, a branched α-olefin, a vinylic olefin, an internal straight-chain olefin, an internal branched olefin, or any mixture of these.

The straight-chain and branched α-olefins or internal olefins used in the preparation are prepared either by oligomerization or dimerization of olefin monomers, by dehydrogenation of alcohols to olefins, by isomerization of α-olefins, by a metathesis reaction, or the olefin may have been prepared by pyrolysis, at a temperature of 300-600 degrees, from a recycled high-molecular-weight polyethylene waste or other plastic.

The mixture of internal olefins and α-olefins used for the preparation may be prepared, for example by oligomerizing a mixture of 1- and 2-butene, i.e. the so-called raffinate II stream, from which isobutene has been removed by reacting it with methanol. The oligomerization may be carried our by using a batch, semi-batch or continuous-working process. The oligomerization catalyst used may be a homogenous Lewis acid catalyst, such as a BF₃ alcohol or BF₃ acid complex or an Al₃Cl₃-HCl, Al₃Cl₂CH₂CH₃ or Al₃Cl₃ aromatic catalyst. The heterogenous oligomerization catalyst used, for its part, may be a synthetic aluminum silica or zeolite.

The mixture of internal and α-olefins may also be prepared by dehydrogenating one or more branched alcohols having 6-10 carbon atoms and by dimerizing the obtained olefin by using above-mentioned catalysts. Some suitable alcohols are, for example, 2-propylheptanol and 2-ethylhexanol.

The olefin may also be a polymer prepared from an olefinic hydrocarbon, i.e. a polyolefin, and in this case, for example, an ASA suitable as a dispersing agent for lubricants is prepared from a polyolefin having a molecular weight of approx. 900-1500.

Below, examples are described of the end uses of ASAs prepared from olefins of various structures and molecular weights, but these examples are in no way tied with the end use of ASAs prepared in accordance with the invention, and they do not restrict the end use of ASAs prepared in accordance with the invention. The structure and molar mass of the olefin used in the preparation depends, for its part, on the intended end use of the ASA, for example so that ASAs prepared from olefins having a molar mass of 140-308 g/mol are used in hydrophobic sizing of paper and in epoxy resin setting agents. ASAs prepared from olefins having a molar mass of 224-490 g/mol are used as corrosion inhibitors and as additives in fuels and lubricants. ASA prepared from lighter-weight olefins, having a molar mass of <140 g/mol, are used as additives in foods. ASA prepared from C₁₄-C₂₀ olefins can also be used in leather treatment agents. The amide, imide and ester derivatives of ASA prepared from C₁₀-C₃₅ olefins can be used as additives in lubricant oils and also as anticorrosive agents.

ASA is prepared at a temperature of 150-250 °C in either a batch reactor or a semi-batch reactor. When ASA is prepared in a semi-batch reactor, MHA is added, to the olefin and any additive according to the invention, in several batches or as a continuous feed at either an even or variable rate, as disclosed, for example, in the claims of patent publication US 4 414 397. It is recommendable to use a reaction temperature of 180-230 °C to improve the efficiency of the reaction. The reaction period may be 0.5-24 hours. The ratio of olefin to MHA may vary within 0.1-5.0/1 (mol/mol), depending on the reaction temperature, reaction period, reaction type, and additive used. The amount of additive in the reaction mixture is for its part also dependent on the reaction temperature, reaction period, reaction type, and the additive used, so that the additive according to the invention is added to the reaction mixture in concentrations in accordance with the invention. Finally any unreacted initial substances are removed from the reaction mixture by vacuum distillation, and an end product (ASA) which is so pure that it need not be purified before use is obtained with a good yield.

In the following examples, additive mixtures according to the invention for use in the preparation of ASA are presented and compared with individual additives and with prior known additives. The results of the preparation examples are shown in the following tables, and the following acronyms are used in them: BHT = 2,6-ditertbutyl-4-methylphenol; FT = phenothiazine; HK = hydroquinone; TiBu = titanium butoxide; FeCl₃ = iron chloride; BHKDA = tertbutylhydroquinonediacetate.

In the experiments in accordance with Examples 1-12, one initial material used is straight-chain C₁₂ α-olefin and the other a maleic anhydride. In addition, an additive mixture according to the invention, individual additives, or prior known additives are added to the reaction mixture in order to improve the yield of ASA and to reduce losses of the initial materials. The additives/additive mixtures used in the examples are shown in Table 1. The reactions were performed in a 300 ml Parr pressure reactor. First the olefin, next the MHA and last the additive/s were weighed into the reactor. The reactor was heated to 220 °C and was maintained at that temperature for 4 hours. After the reaction period the reactor was cooled by means of water circulation to 60 °C, and the reactor was opened. The formed reaction mixtures were analyzed by liquid chromatography by using an RI detector. The yield of ASA of the theoretical maximum and the losses of initial materials were calculated. The results obtained are shown in Table 2.

**Table 1.**

| Additives used in Examples 1-12. | | |
|---|---|---|
| Example | Additive | Reference patent |
| 1 | - | - |
| 2 | Titanium butoxide | US 4 599 433 |
| 3 | Aluminum acetylacetonate | US 4 761 488 |
| 4 | Iron chloride | US 4 431 825 |
| 5 | 2,6-diterbutyl-4-methylphenol | US 3 412 111 |
| 6 | Hydroquinone | US 3 412 111 |
| 7 | 4-methoxyphenol | EP 359 316 |
| 8 | 4,4'-methylenebis-(2,6-diterbutylphenol) | US 3 476 774 |
| 9 | Phenothiazine + 2,6-diterbutyl-4-methylphenol | |
| 10 | Phenothiazine + 4,4'-methylenebis-(2,6-diterbutylphenol) | |
| 11 | Phenothiazine + 4-methoxyphenol | |
| 12 | Phenothiazine + hydroquinone | |

**Table 2.**

| Amounts of materials weighed into the Parr pressure reactor and results from Examples 1-12. | | | | | | |
|---|---|---|---|---|---|---|
| Example | α-C₁₂ [g] | MHA A [g] | Additive [g] | ASA yield [%] | Olefin loss [%] | MHA loss [%] |
| 1 | 85.4 | 40.1 | - | 36.7 | 34.9 | 63.3 |
| 2 | 89.4 | 41.3 | 0.74 | 50.3 | 21.5 | 47.1 |
| 3 | 85.3 | 40.4 | 0.9 | 58.6 | 13.9 | 38.7 |
| 4 | 87.2 | 41.0 | 0.4 | 54.0 | 47.0 | 30.1 |
| 5 | 86.5 | 40.8 | 1.0 | 61.2 | 18.0 | 35.1 |
| 6 | 85.3 | 40.4 | 0.9 | 69.1 | 11.0 | 27.3 |
| 7 | 82.1 | 40.2 | 0.36 | 69.4 | 10.2 | 27.8 |
| 8 | 91.1 | 42.2 | 1.04 | 66.7 | 9.9 | 29.7 |
| 9 | 84.3 | 41.6 | 0.29 + 0.32 | 71.9 | 8.1 | 22.4 |
| 10 | 84.3 | 42.0 | 0.26 + 0.37 | 70.7 | 8.2 | 24.0 |
| 11 | 83.0 | 41.9 | 0.36 + 0.17 | 71.5 | 8.6 | 23.0 |
| 12 | 101.0 | 47.7 | 0.40 + 0.26 | 74.3 | 7.2 | 19.8 |

In Examples 13 (reference US 3 412 111) and 14, the olefinic initial material used was a C₁₃-C₁₈ internal straight-chain olefin. Table 3 shows the amounts of initial materials, the additives and their amounts, the yield of ASA in wt.%, the reaction temperature and the reaction period used in Examples 13 and 14.

**Table 3.**

| Examples 13 and 14. | | | | | | | |
|---|---|---|---|---|---|---|---|
| Example | Olefin [g] | MHA [g] | Additive | Additive amount [g] | ASA yield wt. % | Temperature [°C] | Time [h] |
| 13 | 100.2 | 52.0 | BHT | 0.3 | 53.1 | 190 | 6 |
| 14 | 100.3 | 51.5 | FT + HK | 0.3 + 0.3 | 61.7 | 190 | 6 |

In Examples 15 (reference US 4 599 433) and 16, the olefinic initial material used was an olefin which had been prepared by oligomerizing a mixture of 1-butene and 2-butene (so-called raffinate II stream) by using a synthetic aluminum silica catalyst. The fraction used was a C₁₄-C₁₆ fraction separated by distillation. Table 3 shows the amounts of the initial materials, the additives and their amounts, the yield of ASA in wt.%, the reaction temperature and the reaction period used in Examples 15 and 16.

**Table 4.**

| Examples 15 and 16. | | | | | | | |
|---|---|---|---|---|---|---|---|
| Example | Olefin [g] | MHA [g] | Additive | Additive amount [g] | ASA yield wt. % | Reaction temperature [°C] | Reaction time [h] |
| 15 | 101.2 | 51.6 | TiBu | 0.5 | 58.2 | 210 | 4 |
| 16 | 102.1 | 52.1 | FT + TBHKDA | 0.3 + 0.3 | 68.5 | 210 | 4 |

In Examples 17 (reference US 4 431 825) and 18, the olefinic initial material used was a mixture of C₁₄-C₁₈ α-olefins and C₁₅-C₁₈ internal olefins. Table 5 shows the amounts of the initial materials, the additives and their amounts, the yield of ASA in wt.%, the reaction temperature and the reaction period used in Examples 17 and 18.

**Table 5.**

| Examples 17 and 18. | | | | | | | |
|---|---|---|---|---|---|---|---|
| Example | Olefin [g] | MHA [g] | Additive | Additive amount [g] | ASA yield wt. % | Reaction temperature [°C] | Reaction time [h] |
| 17 | 100.7 | 51.2 | FeCl₃ | 0.5 | 58.5 | 210 | 4 |
| 18 | 100.5 | 50.8 | FT + TBHKDA | 0.3 + 0.3 | 65.9 | 210 | 4 |

In Examples 19 and 20, the olefinic initial material was an olefin which had been obtained from the pyrolysis of polyethylene and from which the fraction containing C₁₂-C₁₈ had been separated by distillation. Table 6 shows the amounts of the initial materials, the additives and their amounts, the yield of ASA in wt.%, the reaction temperature and the reaction period used in Examples 19 and 20.

**Table 6.**

| Examples 19 and 20. | | | | | | | |
|---|---|---|---|---|---|---|---|
| Example | Olefin [g] | MHA [g] | Additive | Additive amount [g] | ASA yield wt. % | Reaction temperature [°C] | Reaction time [h] |
| 17 | 103.5 | 52.1 | BHT | 0.5 | 33.2 | 210 | 4 |
| 18 | 104.2 | 51.2 | FT + BHT | 0.3 + 0.3 | 36.9 | 210 | 4 |

## Claims

1. A process for the preparation of alkenyl succinic anhydride by reacting an olefinically unsaturated hydrocarbon and a maleic anhydride at a temperature of 150-250°C, **characterized in that** a mixture of an aromatic compound which contains nitrogen and sulfur and a phenolic antioxidant or a derivative thereof is used as an additive in the reaction.

2. A process according to Claim 1, **characterized in that** each additive is used in an amount of 0.01-5.0 mol % of the amount of the maleic anhydride.

3. A process according to Claim 1 or Claim 2,
**characterized in that** the aromatic compound which contains nitrogen and sulfur is phenothiazine.

4. A process according to any preceding Claim, **characterized in that** the phenolic antioxidant is 2,6-ditertbutyl-4-methylphenol, tertbutylhydroquinonediacetate, hydroquinone, 4,4'-methylenebis-(2,6-ditertbutylphenol) or hydroquinonemonomethylether.

5. A process according to any preceding Claim, **characterized in that** the weight ratio of the aromatic compound which contains nitrogen and sulfur to the phenolic antioxidant or derivative thereof is 0.1-10.

6. A process according to Claim 3, **characterized in that** the weight ratio of the phenothiazine to the phenolic antioxidant or derivative thereof is 0.25-5.

7. A process according to any preceding Claim, **characterized in that** in the reaction mixture the molar ratio of the initial materials is 0.1-5.0 mol of olefinic hydrocarbon to 1.0 mol of maleic anhydride.

8. A process according to any preceding Claim, **characterized in that** the olefinically unsaturated hydrocarbon is a straight-chain α-olefin or a straight-chain or branched internal olefin, or a mixture thereof.

9. A process according to Claim 8, **characterized in that** the length of the carbon chain in the olefin is 6-20 carbon atoms or the olefin is a polyolefin having a molecular weight of 900-1500.

10. A process according to any preceding Claim, **characterized in that** the reaction is performed in a batch reactor or a semi-batch reactor.

11. A process according to Claim 10, **characterized in that** in a semi-batch reactor the maleic anhydride is added in several batches or as a continuous feed having an even or variable feeding speed.

## Patentansprüche

1. Verfahren zur Herstellung eines Alkenylbernsteinsäureanhydrids durch Umsetzen von einem olefinisch ungesättigten Kohlenwasserstoff und Maleinsäureanhydrid bei einer Temperatur von 150 bis 250°C, **dadurch gekennzeichnet, daß** eine Mischung einer aromatischen Verbindung, welche Stickstoff und Schwefel enthält, und eines phenolischen Antioxidationsmittels oder eines Derivats hiervon in der Reaktion als Additiv verwendet wird.

2. Verfahren gemäß Anspruch 1, **dadurch gekennzeichnet, daß** jedes Additiv in einer Menge von 0,01 bis 5,0 Mol-% der Menge des Maleinsäureanhydrids verwendet wird.

3. Verfahren gemäß Anspruch 1 oder Anspruch 2, **dadurch gekennzeichnet, daß** die aromatische Verbindung, welche Stickstoff und Schwefel enthält, Phenothiazin ist.

4. Verfahren gemäß einem der vorangehenden Ansprüche, **dadurch gekennzeichnet, daß** das phenolische Antioxidationsmittel 2,6-Di-tert-butyl-4-methylphenol, tert-Butylhydrochinondiacetat, Hydrochinon, 4,4'-Methylen-bis-(2,6-di-tert-butylphenol) oder Hydrochinonmonomethylether ist.

5. Verfahren gemäß einem der vorangehenden Ansprüche, **dadurch gekennzeichnet, daß** das Gewichtsverhältnis der aromatischen Verbindung, welche Stickstoff und Schwefel enthält, zu dem phenolischen Antioxidationsmittel oder Derivat hiervon 0,1 bis 10 beträgt.

6. Verfahren gemäß Anspruch 3, **dadurch gekennzeichnet, daß** das Gewichtsverhältnis des Phenothiazins zu dem phenolischen Antioxidationsmittel oder Derivat hiervon 0,25 bis 5 beträgt.

7. Verfahren gemäß einem der vorangehenden Ansprüche, **dadurch gekennzeichnet, daß** in der Reaktionsmischung das Molverhältnis der Ausgangsstoffe 0,1 bis 5,0 mol olefinischer Kohlenwasserstoff zu 1,0 mol Maleinsäureanhydrid beträgt.

8. Verfahren gemäß einem der vorangehenden Ansprüche, **dadurch gekennzeichnet, daß** der olefinisch ungesättigte Kohlenwasserstoff ein geradkettiges α-Olefin oder ein geradkettiges oder verzweigtes inneres Olefin, oder eine Mischung davon ist.

9. Verfahren gemäß Anspruch 8, **dadurch gekennzeichnet, daß** die Länge der Kohlenstoff-Kette im Olefin 6 bis 20 Kohlenstoffatome beträgt oder das Olefin ein Polyolefin mit einem Molekulargewicht von 900 bis 1500 ist.

10. Verfahren gemäß einem der vorangehenden Ansprüche, **dadurch gekennzeichnet, daß** die Reaktion in einem Batch-Reaktor oder einem Halbbatch-Reaktor durchgeführt wird.

11. Verfahren gemäß Anspruch 10, **dadurch gekennzeichnet, daß** in einem Halbbatch-Reaktor das Maleinsäureanhydrid in mehreren Batches oder als kontinuierliche Einspeisung mit einer gleichmäßigen oder variablen Einspeisungsgeschwindigkeit zugegeben wird.

## Revendications

1. Procédé de préparation d'un anhydride alcénylsuccinique par réaction d'un hydrocarbure à insaturation oléfinique et d'un anhydride maléique à une température de 150 à 250°C, **caractérisé en ce qu'**on utilise comme additif dans la réaction un mélange d'un composé aromatique qui contient de l'azote et du soufre et d'un anti-oxydant phénolique ou d'un dérivé de ce dernier.

2. Procédé selon la revendication 1, **caractérisé en ce que** chaque additif est utilisé en une quantité de 0,01 à 5,0 % en mole par rapport à la quantité de l'anhydride maléique.

3. Procédé selon la revendication 1 ou 2, **caractérisé en ce que** le composé aromatique qui contient de l'azote et du soufre est la phénothiazine.

4. Procédé selon l'une quelconque des revendications précédentes, **caractérisé en ce que** l'anti-oxydant phénolique est le 2,6-di-tert-butyl-4-méthylphénol, le diacétate de tert-butylhydroquinone, l'hydroquinone, le 4,4'-méthylènebis(2,6-di-tert-butylphénol) ou l'éther monométhylique de l'hydroquinone.

5. Procédé selon l'une quelconque des revendications précédentes, **caractérisé en ce que** le rapport en poids du composé aromatique qui contient de l'azote et du soufre à l'anti-oxydant phénolique ou un dérivé de ce dernier est de 0,1 à 10.

6. Procédé selon la revendication 3, **caractérisé en ce que** le rapport en poids de la phénothiazine à l'anti-oxydant phénolique ou un dérivé de ce dernier est de 0,25 à 5.

7. Procédé selon l'une quelconque des revendications précédentes, **caractérisé en ce que**, dans le mélange réactionnel, le rapport en moles entre les matières de départ est de 0,1 à 5,0 moles d'hydrocarbure oléfinique pour 1,0 mole d'anhydride maléique.

8. Procédé selon l'une quelconque des revendications précédentes, **caractérisé en ce que** l'hydrocarbure à insaturation oléfinique est une α-oléfine à chaîne droite ou une oléfine interne à chaîne droite ou ramifiée, ou un mélange de celles-ci.

9. Procédé selon la revendication 8, **caractérisé en ce que** la longueur de la chaîne carbonée de l'oléfine est de 6 à 20 atomes de carbone, ou encore l'oléfine est une polyoléfine ayant une masse moléculaire de 900 à 1500.

10. Procédé selon l'une quelconque des revendications précédentes, **caractérisé en ce que** la réaction est mise en oeuvre dans un réacteur discontinu ou semi-discontinu.

11. Procédé selon la revendication 10, **caractérisé en ce que**, dans un réacteur semi-discontinu, l'anhydride maléique est introduit en plusieurs lots, ou sous forme d'une alimentation continue ayant un débit d'alimentation fixe ou variable.
